# EUROPEAN PATENT APPLICATION

(11) **EP 2 559 758 A1**
(43) Date of publication of application: **20.02.2013**
(21) Application number: 11177763.7
(22) Date of filing: 17.08.2011
(51) Int. Cl.: C12N 9/02

(54) **Modified glyceraldehyde-3-phosphate dehydrogenase from corynebacterium glutamicum and uses thereof**

(71) Applicant: Technische Universität Hamburg-Harburg, 21073 Hamburg (DE)
(72) Inventor: Zeng, An-Ping, 21224 Rosengarten (DE); Chen, Zhen, 21073 Hamburg (DE); Rappert, Sugima, 22089 Hamburg (DE)
(74) Representative: UEXKÜLL & STOLBERG

(57) **Abstract**

The present invention relates to enzymatically active glyceraldehyde-3-phosphate dehydrogenase enzymes, which have been modified in their primary structure to alter their coenzyme specificity. In particular, the modified enzymes of the invention exhibit an improved specificity for the coenzyme NADPH which is associated with several advantages in terms of their applicability in biotechnological processes. The invention also provides methods for producing an amino acid, in particular L-lysine or L-threonine, which make use of the modified glyceraldehyde-3-phosphate dehydrogenase enzyme. The invention also relates to the use of the modified glyceraldehyde-3-phosphate dehydrogenase enzyme for biotechnological production processes.

## Description

The present invention relates to enzymatically active glyceraldehyde-3-phosphate dehydrogenase enzymes, which have been modified by certain amino acid exchanges to alter their coenzyme specificity. In particular, the modified enzymes of the invention exhibit an improved specificity for the coenzyme NADP which is associated with several advantages in terms of their applicability in biotechnological processes. The invention also provides methods for producing an amino acid, in particular L-lysine or L-threonine, which make use of the modified glyceraldehyde-3-phosphate dehydrogenase enzyme. The invention also relates to the use of the modified glyceraldehyde-3-phosphate dehydrogenase enzyme for biotechnological production processes.

### BACKGROUND OF THE INVENTION

Glyceraldehyde 3-phosphate dehydrogenase (GAPDH) is an important enzyme in the energy metabolism and is indispensible for a glycolytically active organism. Physiologically, GAPDH plays a role, e.g., in gluconeogenesis, a process in which glucose is produced from precursor molecules such as pyruvate, oxal-acetate, lactate or dihydroxyacatonphosphate. The majority of GAPDH subtypes catalyzes the conversion of glyceraldehyde 3-phosphate to 1,3-bisphosphoglycerate and simultaneously reduces the coenzyme NAD(P)⁺ to NAD(P)H. For each mol glucose which is converted to 1,3-bisphosphoglycerate, 2 mol NAD(P)H are generated during glycolysis.

Three types of endogenous GAPDH enzymes have been described to date, GapA (EC number 1.2.1.12), GapB (EC number 1.2.1.13) and GapN (EC number 1.2.1.9). These enzymes are encoded by the *gapA, gapB* and *gapN* genes, respectively. GapA and GapB are phosphorylating enzymes, whereas GapN does not provide for a phosphorylation. The three GAPDH-subtypes require different coenzymes. GapA is NAD dependent, GapB accepts both NAD and NADP with a preference for NADP, and GapN is NADP dependent. The GAPDH subtypes also differ in their physiological function. GapA is catalytically active in the conversion of glyceraldehyde 3-phosphate to 1,3-bisphosphoglycerate during glycolysis as well as in the reverse reaction during gluconeogenesis, while GapB is only involved in the forward reaction during gluconeogenesis (Fillinger et al. (2000), J. Biol. Chem., 275:14031-14037). GapN catalyzes the irreversible oxidation of glyceraldehyde 3-phosphate to 3-phosphoglycerate without generation of ATP and shows only a low homology to the phosphorylating GAPDHs GapA and GapB (Habenicht et al. (1994), J. Mol. Biol. 237:165-71).

GapA or GapB are used in the industrial production of many important compounds, as they are able to provide a source of reducing equivalents, i.e. they are able to transfer the equivalent of one electron in redox reactions by means of their coenzymes NAD⁺ or NADP⁺. NADP is a derivative of NAD, which is phosphorylated at the C2' in the ribose ring. Both coenzymes have separate functions in the metabolism. NAD is catabo-lically produced in the citrate cycle and during glycolysis, and it is oxidized in the respiratory chain to produce ATP. In contrast, NADP serves predominantly as a reducing agent in anabolic processes.

NADPH as reducing equivalent is involved in many important bioprocesses for the synthesis of industrially important compounds, such as sugars or amino acids, and its supply is often a limiting factor for achieving high yield and productivity. Such industrially important compounds are often produced by cultivation of microorganisms, as these are often a cheaper and more efficient alternative to conventional chemical synthesis. In the cytosol of microorganisms, NADPH is provided primarily by enzymes of the pentose phosphate pathway (PPP), including glucose-6-phosphate dehydrogenase and 6-phosphogluconate dehydrogenase. The NADP-dependent isocitrate dehydrogenase and malic enzyme in the tricarboxylic acid cycle may also be a source of NADPH to some extent in some microorganisms (Marx et al. (1997), Biotechnol. Bioeng. 56:168-180).

It has been shown that NADPH is one of the limiting factors in the production of L-lysine from glucose in an industrial scale in *Corynebacterium glutamicum* (Becker et al. (2005), Appl. Environ. Microbiol. 71(12):8587-96). Other compounds whose production requires NADPH are, e.g., sugars such as glucose, fructose and sucrose, amino acids, such as methionine (Kumar et al. (2003), Process. Biochem. 38:1165-1171), isoleucine (Morbach et al. (1996), Appl. Environ. Microbiol. 62:4345-4351), or aromatic amino acids (Ikeda and Katsumata. (1999), Appl. Environ. Microbiol. 65:2497-2502; Ikeda et al.(1999), Appl. Microbiol. Biotechnol. 51:201-206), fatty acids (Gema et al. (2002), Appl. Microbiol. Biotechnol. 58:303-307), and products derived from the pentose phosphate pathway (PPP), such as vitamin B₂ (Stahmann et al. (2000), Appl. Microbiol. Biotechnol. 53:509-516), and nucleotides (Abbouni et al. (2004), Arch. Microbiol. 182:119-125; Kamada et al. (2001), Appl. Microbiol. Biotechnol. 56:710-717).

Overexpression of NADP-dependent enzymes such as glucose-6-phosphate dehydrogenase has been reported to improve lysine production in *C*. *glutamicum* (Becker et al. (2007), J. Biotechnol. 132:99-109). However, it was also found that rough changes in the bacterial metabolism often lead to severe growth defects (Becker et al. (2005), Appl. Environ. Microbiol. 71(12):8587-96; Marx et al. (2003), J. Biotechnol. 104:185-197). Therefore, it would be desirable to identify means that could provide for a supply of the reducing equivalent NADPH to increase productivity and yield of industrial bioprocesses without disturbing the metabolism of the host cell.

In view of the fact that GapA - unlike GapB - is an enzyme that is involved in glycolysis, it is a potential source of reduced coenzyme (Omumasaba et al. (2004), J. Mol. Microbiol. Biotechnol. 8:91-103). However, GapA is strictly specific for NAD. It would therefore be desirable to change the coenzyme-specificity of the GapA enzyme from NAD to NADP in order to increase the product yields of NADP-dependent syntheses such as the production of L-lysine.

Different attempts have been made in the prior art to switch the coenzyme specificity of GapA of *Bacillus stearothermophilus* from NAD towards NADP. These attempts focused on the acidic residue Asp32 and residues at the subunit interface (Clermont et al. (1993), Biochemistry. 32:10178-10184). It was shown that a replacement of the amino acids Leu187 and Pro188 at the subunit interface by Ala and Ser, respectively, conferred activity with the coenzyme NADP (Corbier et al. (1990), Biochemistry, 29:7101-7106). However, a full reversal of coenzyme specificity, in terms of having a mutant enzyme which is catalytically as efficient with NADP as the wildtype with NAD, has not been achieved so far.

The present invention provides enzymatically active GAPDH enzymes of the GapA subtype which have been modified by certain amino acid exchanges to alter their coenzyme specificity from NAD towards NADP. Specifically, some of the enzymes provided by the present invention exhibit a complete reversal of the coenzyme specificity which renders them particularly suitable for being used in biotechnological production processes, e.g., for the production of amino acids, such as L-lysine or L-threonine.

### DISCLOSURE OF THE INVENTION

In a first aspect, the present invention relates to a modified glyceraldehyde-3-phosphate dehydrogenase (GAPDH) enzyme which is enzymatically active and has an increased specificity to coenzyme NADP relative to the corresponding naturally occurring enzyme. The modified GAPDH enzyme has the following characteristics:
(a) it has an amino acid sequence which is at least 50% identical to the amino acid sequence depicted in SEQ ID NO:1; and
(b) it comprises amino acid replacements (relative to the naturally occurring enzyme) in positions that correspond to amino acids 35 and 36 of SEQ ID NO:1.

The invention is based on the finding that the specificity of the NAD-dependent GAPDH enzyme of *C*. *glutamicum* to NADP can be modified by certain amino acid exchanges in its naturally occurring amino acid sequence. The naturally occurring NAD-dependent GAPDH enzyme of *C*. *glutamicum* is depicted in SEQ ID NO:1. Specifically, it has been found that a combination of amino acid exchanges in the phosphate binding site of the enzyme is effective in shifting the specificity of the enzyme from NAD to NADP. As shown in the below examples, replacement of amino acids 35 and 36 of the native sequence of the NAD-dependent GAPDH enzyme of *C*. *glutamicum* resulted in a mutant GAPDH enzyme which was shown to be catalytically active not only with NAD, but also with NADP. Such a modified enzyme is particularly suitable for use in industrial biosynthetic processes, such as the production of amino acids like L-lysine or L-threonine.

As used in the following, a "GAPDH" or "GAPDH enzyme" refers to an NAD-dependent enzyme of the Enzyme Classification number EC 1.2.1.12. Similarly, a "modified GAPDH" or "modified GAPDH enzyme" refers to an enzyme of the EC 1.2.1.12 category that comprises a primary structure in which at least two amino acids have been replaced compared to the amino acid sequence of the naturally occurring enzyme, resulting in an enzyme that has an increased specificity to coenzyme NADP relative to the corresponding naturally occurring enzyme. The modified GAPDH can make use of NADP as the only coenzyme, or it can be able to utilize both NAD and NADP. Preferably, the modified GAPDH of the invention has been derived from a bacterial enzyme. The modified GAPDH is enzymatically active which means that it catalyzes the conversion of glyceraldehyde 3-phosphate to 1,3-bisphosphoglycerate.

In one preferred aspect, the modified GAPDH enzyme of the invention is the *C*. *glutamicum* enzyme depicted in SEQ ID NO:1 which has been modified by replacing at least the native amino acids Asp35 and Leu36. These amino acids form the N-terminal part of a conserved sequence motif, Asn-Asp-Leu-Thr/Leu-Asp (SEQ ID NO:16), which is found in the primary structure of all bacterial NAD-dependent GAPDH enzymes. The conserved sequence is located within a phosphate binding site which is crucial for binding of coenzyme NAD. A site-directed mutagenesis which results in the replacement of Asp35 and Leu36 in the *C*. *glutamicum* enzyme enables the enzyme to use both coenzymes NAD and NADP with essentially the same specificity (see Table 1). If this modification is combined with an additional replacement at position 37 or 192 (or replacements in both positions), an almost completely reversed selectivity of the enzyme is achieved, which means that the formerly NAD-dependent GAPDH has become almost completely NADP-dependent.

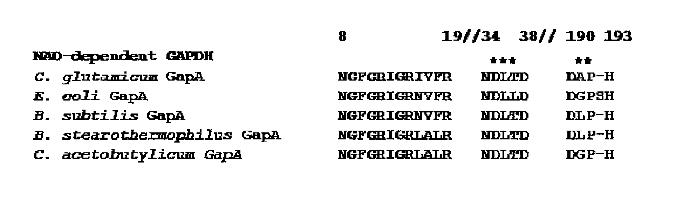

It must be understood that the concept of the present invention is clearly not limited to the particular *C*. *glutamicum* enzyme of SEQ ID NO:1. Instead, it is broadly applicable to GAPDH enzymes from other species which produce a GAPDH enzyme that is closely related to the enzyme from *C*. *glutamicum.* As shown in Figure 1, the GAPDH enzymes from various bacteria share a high degree of sequence identity on the polypeptide level with the GAPDH of *C*. *glutamicum.* Notably, these enzymes exhibit the conserved phosphate-binding amino acid motif which is located in the N-terminal part of the polypeptide. It can therefore be expected that amino acid replacements at positions which correspond to those identified in the *C*. *glutamicum* enzyme will likewise result in an increased specificity of these enzymes towards NADP. Accordingly, the invention includes also modified GAPDH enzymes which have been derived from naturally occurring enzymes that are closely related to the *C*. *glutamicum* GAPDH depicted in SEQ ID NO:1.

A modified GAPDH enzyme of the invention which has been derived from a species other than *C*. *glutamicum* will exhibit an amino acid sequence which is at least 50% identical to the amino acid sequence depicted in SEQ ID N:1, and it will be characterized by amino acid replacements (relative to the naturally occurring enzyme) in positions that correspond to amino acids 35 and 36 of SEQ ID NO:1. The amino acid identity of the modified GAPDH with SEQ ID NO:1 will preferably be at least about 55%, 60%, 70%, 75%, 80%, 85%, 90%, 91%, 92%, 93%, 94%, 95%, 96%, 97%, 98% or 99% when compared in an optimal alignment, for example, by the program BESTFIT using standard parameters. A sequence identity of 90% means that 90% of the amino acids of an analyzed amino acid sequence stretch are identical to the sequence of the reference amino acid sequence depicted in SEQ ID NO:1. Methods and computer programs for determining amino acid sequence identity are well known in the art. It is preferred that the recited amino acid sequence identity occurs over an amino acid sequence stretch of at least 50, 100, 200, 250, 300 or 330 amino acids. More preferably, the recited sequence identity occurs over the complete length of the polypeptide, i.e. 334 amino acids.

A modified GAPDH enzyme of the invention which originates from a species other than *C*. *glutamicum* will preferably also exhibit the conserved consensus motif GXGXXG (SEQ ID NO:17), wherein X can be any amino acid; this motif is indicative for a Rossmann fold of a NAD binding enzyme.

The two residues which are to be replaced according to the present invention are located at positions 35 and 36 in the GAPDH sequence of *C*. *glutamicum* in SEQ ID NO:1. These positions are part of the conserved phosphate binding motif, and their numbering may differ depending on the species from which the GAPDH enzyme to be modified originates. For example, the amino acid motif Asn-Asp-Leu-Thr-Asp (SEQ ID NO:16) is located at positions 31-35 of the GAPDH enzyme of *B*. *stearothermophilus,* which means that the positions which correspond to amino acids 35 and 36 in the GAPDH sequence of *C*. *glutamicum* are amino acids 32 and 33 in the enzyme of *B. stearothermophilus.* The skilled person will readily be able to identify the amino acid which correspond to amino acids 35 and 36 of SEQ ID NO:1 in the amino acid sequence of GAPDH enzymes from other organisms. Preferably, the modified GAPDH enzymes of the present invention are derived from naturally occurring enzymes which comprise the amino acid motif Asn-Asp-Leu-Thr/Leu-Asp in their unmodified primary structure.

Generally, the amino acid positions that correspond to amino acids 35 and 36 of SEQ ID NO:1 may be substituted with any type of alternative amino acid as long as the resulting GAPDH enzyme exhibits an increased specificity for NADP compared to the corresponding naturally occurring enzyme. For example, the amino acids in these positions can be replaced by any proteinogenic or non-proteinogenic amino acid. Preferably, the amino acids in the GAPDH enzyme are replaced by a proteinogenic amino acid. As used herein, proteinogenic amino acids are those 23 amino acids which are regularly found in naturally occurring polypeptides, i.e. isoleucine (Ile), alanine (Ala), leucine (Leu), asparagine (Asn), lysine (Lys), aspartic acid (Asp), methionine (Met), cysteine (Cys), phenylalanine (Phe), glutamic acid (Glu), threonine (Thr), glutamine (Gln), tryptophan (Trp), glycine (Gly), valine (Val), proline (Pro), serine (Ser), tyrosine (Tyr), arginine (Arg), histidine (His), selenocysteine, selenomethionine, and pyrrollysine. Preferably, the amino acids used for the replacement are L-amino acids. However, also D-amino acids may be used.

In addition to proteinogenic amino acids, the amino acids used for replacing the amino acids in the naturally occurring enzyme can be non-proteinogenic amino acids, i.e. amino acids which are not found in naturally occurring polypeptides. These non-proteinogenic amino acids include, for example, α-aminoadipic acid, β-aminoadipic acid, α-aminobutyric acid, α-aminoisobutyric acid, β-alanine, 4-aminobutyric acid, 5-aminovaleric acid, 6-aminohexanoic acid, 8-aminooctanoic acid, 9-aminononanoic acid, 10-aminodecanoic acid, 12-aminododecanoic acid, α-aminosuberic acid, β-cyclohexylalanine, citrulline, dehydroalanine, α-cyclohexylglycine, propargylglycine, pyroglutamic acid, 4-benzoylphenylalanine, δ-hydroxylysine, 4-hydroxyproline, allo-isoleucine, lanthionine (Lan), norleucine, norvaline, ornithine, phenylglycin, pipecolic acid, sarcosine, 1,2,3,4-tetrahydro-isochinoline-3-carboxylic acid, allo-threonine, thiazolidine-4-carboxylic acid, γ-aminobutyric acid (GABA), iso-cysteine, diaminopropionic acid, 2,4-diaminobutyric acid, 3,4-diaminobutyric acid, biphenylalanine and 4-fluoro-phenylalanine.

Also included by the term "non-proteinogenic amino acids" are derivatives of the above-mentioned proteinogenic amino acids wherein a side-chain has been modified, for example, by a methylene group, thereby providing e.g. homomethionine, homoserine, homoproline, homothreonine, homotryptophane, homotyrosine, homohistidine and homolysine.

However, it is particularly preferred according to the invention that the positions which correspond to amino acids 35 and 36 in the GAPDH sequence of *C*. *glutamicum* are replaced by a proteinogenic amino acid. In the enzyme of *C*. *glutamicum,* the carboxylate group of Asp35 was found to form strong hydrogen bonds with the 2' and 3'-hydroxyl groups of the adenosine ribose ring of NAD. In contrast, the 2' phosphate group of NADP cannot bind, presumably due to electrostatic repulsion. Therefore, the Asp residue at position 35 in the GAPDH enzyme of *C*. *glutamicum* (or at a position that corresponds to Asp35 of SEQ ID NO:1 insofar as a GAPDH from another organism is concerned) is preferably replaced by a small and non-charged amino acid which would eliminate the potential electrostatic repulsion. Suitable amino acids for the replacement include Gly, Ala, Val, Leu, Ile, or Pro. In a particularly preferred embodiment, the Asp residue at position 35 in the GAPDH enzyme of *C*. *glutamicum* (or at a position that corresponds to Asp 35 of SEQ ID NO:1 insofar as a GAPDH from another organism is concerned) is replaced by a Gly or Ala residue.

The amino acid at position 36 is supposed to interact with NADP in order to stabilize binding of this coenzyme. Preferably, the amino acid at position 36 of SEQ ID NO:1 (or at a position that corresponds to amino acid 36 of SEQ ID NO:1) can be modified by replacing the Leu residue by hydrophilic or positively charged amino acid, such as Ser, Thr, Arg, His, or Lys. In a particularly preferred embodiment, the amino acid at position 36 of SEQ ID NO:1 (or at a position that corresponds to amino acid 36 of SEQ ID NO:1 insofar as a GAPDH from another organism is concerned) is replaced by Thr or Arg.

In an even more preferred embodiment, the Asp residue in the position that corresponds to amino acid 35 of SEQ ID NO:1 has been replaced by Gly, and the Leu residue in the position that corresponds to amino acid 36 of SEQ ID NO:1 has been replaced by Thr. As far as the GAPDH sequence of *C*. *glutamicum* is concerned, this means that the enzyme sequence depicted in SEQ ID NO:1 has been modified such that the modified enzyme has a Gly residue in position 35 and a Thr residue in position 36. In another preferred embodiment, the Asp residue in the position that corresponds to amino acid 35 of SEQ ID NO:1 has been replaced by Ala, and the Leu residue in the position that corresponds to amino acid 36 of SEQ ID NO:1 has been replaced by Thr. For the modified GAPDH enzyme of *C*. *glutamicum,* this means that SEQ ID NO:1 has been modified such that the residue in position 35 is Ala and the residue in position 36 is Thr.

In another preferred embodiment of the invention, the modified GAPDH enzyme which comprises the amino acid replacements referred to above also comprises one or more of the following replacements:
(a) Thr or Leu in the position that corresponds to amino acid 37 of SEQ ID NO:1 has been replaced by another amino acid;
(b) Pro in the position that corresponds to amino acid 192 of SEQ ID NO:1 has been replaced by another amino acid.

The amino acids that can be used for replacing the amino acids occurring in positions 37 and 192 of the native sequence of the enzyme can be any of the proteinogenic or non-proteinogenic amino acids discussed above. Preferably, however, the Thr or Leu residue in the position that corresponds to amino acid 37 of SEQ ID NO:1 is replaced by Lys, Arg or His, while the Pro in the position that corresponds to amino acid 192 of SEQ ID NO:1 is replaced by Ser, Thr or Arg. It has been found in the context of the experiments conducted with the GAPDH enzyme of *C*. *glutamicum* that the combination of amino acids replacement in positions 35/36 with a replacement at position 37 or 192 results in an almost complete reversal of the coenzyme specificity in favor of NADP. While position 37 in the *C*. *glutamicum* enzyme is located in the phosphate binding site, position 192 is located at the interface of the enzyme subunits. Thus, the invention specifically contemplates a modified GAPDH enzyme which has an amino acid sequence which is at least 50% identical to the amino acid sequence depicted in SEQ ID NO:1 and furthermore comprises a combination of amino acid exchanges selected from the group of:
(a) Asp in the position that corresponds to amino acid 35 of SEQ ID NO:1 has been replaced by Gly; Leu in the position that corresponds to amino acid 36 of SEQ ID NO:1 has been replaced by Thr; and Thr or Leu in the position that corresponds to amino acid 37 of SEQ ID NO:1 has been replaced by Lys;
(b) Asp in the position that corresponds to amino acid 35 of SEQ ID NO:1 has been replaced by Gly; Leu in the position that corresponds to amino acid 36 of SEQ ID NO:1 has been replaced by Thr; and Pro in the position that corresponds to amino acid 192 of SEQ ID NO:1 has been replaced by Ser;
(c) Asp in the position that corresponds to amino acid 35 of SEQ ID NO:1 has been replaced by Gly; Leu in the position that corresponds to amino acid 36 of SEQ ID NO:1 has been replaced by Thr; and Thr or Leu in the position that corresponds to amino acid 37 of SEQ ID NO:1 has been replaced by Lys; and Pro in the position that corresponds to amino acid 192 of SEQ ID NO:1 has been replaced by Ser;
(d) Asp in the position that corresponds to amino acid 35 of SEQ ID NO:1 has been replaced by Gly; Leu in the position that corresponds to amino acid 36 of SEQ ID NO:1 has been replaced by Arg; and Thr or Leu in the position that corresponds to amino acid 37 of SEQ ID NO:1 has been replaced by Lys;
(e) Asp in the position that corresponds to amino acid 35 of SEQ ID NO:1 has been replaced by Gly; Leu in the position that corresponds to amino acid 36 of SEQ ID NO:1 has been replaced by Arg; and Pro in the position that corresponds to amino acid 192 of SEQ ID NO:1 has been replaced by Ser; and
(f) Asp in the position that corresponds to amino acid 35 of SEQ ID NO:1 has been replaced by Gly; Leu in the position that corresponds to amino acid 36 of SEQ ID NO:1 has been replaced by Arg; and Thr or Leu in the position that corresponds to amino acid 37 of SEQ ID NO:1 has been replaced by Lys; and Pro in the position that corresponds to amino acid 192 of SEQ ID NO:1 has been replaced by Ser.

In an even more preferred aspect, the invention provides a modified *C*. *glutamicum* GAPDH enzyme which comprises a combination of amino acid exchanges selected from the group of:
(a) Asp in position 35 of SEQ ID NO:1 has been replaced by Gly; Leu in position 36 of SEQ ID NO:1 has been replaced by Thr; and Thr in position 37 of SEQ ID NO:1 has been replaced by Lys;
(b) Asp in position 35 of SEQ ID NO:1 has been replaced by Gly; Leu in position 36 of SEQ ID NO:1 has been replaced by Thr; and Pro in position 192 of SEQ ID NO:1 has been replaced by Ser;
(c) Asp in position 35 of SEQ ID NO:1 has been replaced by Gly; Leu in position 36 of SEQ ID NO:1 has been replaced by Thr; and Thr in position 37 of SEQ ID NO:1 has been replaced by Lys; and Pro in position 192 of SEQ ID NO:1 has been replaced by Ser;
(d) Asp in position 35 of SEQ ID NO:1 has been replaced by Gly; Leu in position 36 of SEQ ID NO:1 has been replaced by Arg; and Thr in position 37 of SEQ ID NO:1 has been replaced by Lys;
(e) Asp in position 35 of SEQ ID NO:1 has been replaced by Gly; Leu in position 36 of SEQ ID NO:1 has been replaced by Arg; and Pro in position 192 of SEQ ID NO:1 has been replaced by Ser; and
(f) Asp in position 35 of SEQ ID NO:1 has been replaced by Gly; Leu in position 36 of SEQ ID NO:1 has been replaced by Arg; and Thr in position 37 of SEQ ID NO:1 has been replaced by Lys; and Pro in position 192 of SEQ ID NO:1 has been replaced by Ser.

The modified GAPDH is characterized by a specificity for coenzyme NADP which is higher than that of the corresponding naturally occurring enzyme. Specificity of the modified GAPDH enzyme in the sense of the present invention is to be understood as the affinity of the GAPDH enzyme for a particular coenzyme. A modified or unmodified GAPDH has specificity for a particular coenzyme if said GAPDH enzyme is capable of binding and reducing the coenzyme in the course of its catalytical activity.

Suitable means for measuring the affinity of a GAPDH enzyme to a coenzyme, such as NAD and NADP, include any method which is able to determine the binding strength between the two binding partners. Such methods include, e.g., the determination of the affinity/association/equilibrium constant Kₐ, the dissociation constant K_{d}, the Michaelis constant Kₘ, the specificity constant k_{cat}/Kₘ and any other method known in the art. The specificity constant k_{cat}/Kₘ is a measure of how efficiently an enzyme converts a substrate into product, wherein k_{cat} is the rate constant of the catalytic reaction and Kₘ is the Michaelis constant.

Suitable methods for determination of above kinetic reaction parameters include assays which allow monitoring of the conversion rate of a reaction, i.e. determination of the coenzyme NAD(P)⁺ and/or NAD(P)H concentration before and after defined reaction times. As GAPDH requires the coenzyme-substrate NAD or NADP as well as the primary substrate glyceraldehyde 3-phosphate or 1,3-bisphosphoglycerate, these methods would have to be carried out in the presence of this primary substrate. Such methods include mixing of the compounds necessary for the reaction, providing suitable reaction conditions and measuring the concentrations of the reaction products or educts after different reaction times. Further, the binding affinity between the coenzyme and GAPDH may also be determined directly by methods which allow the detection of enzyme-bound coenzyme, such as Dynamic Light Scattering (DLS), Surface Plasmon Resonance (SPR), native gel electrophoresis, and the like.

The modified GAPDH enzymes of the present invention exhibit an increased specificity for the coenzyme NADP relative to the corresponding naturally occurring enzyme from which it is derived (i.e. the unmodified GAPDH). This means that the enzyme is able to bind to NADP⁺ and reduce it in the course of its catalytical activity, i.e. the conversion of glyceraldehyde 3-phosphate to 1,3-bisphosphoglycerate, to NADPH. The increased specificity of the modified GAPDH for NADP in that reaction can be measured, for example, by the Michaelis constant Kₘ or by the specificity constant K_{cat}/Kₘ. For example, the specificity constant K_{cat}/Kₘ of the modified GAPDH enzyme is at least about 1x, 2x, 3x, 4x, 5x, 6x, 7x, 8x, 9x, 10x, 15x, 20x or more higher than that of the unmodified enzyme. In absolute terms, the specificity constant K_{cat}/Kₘ of the modified GAPDH enzyme is between 100 and 10000 mM⁻¹ min⁻¹, preferably higher than 500 mM⁻¹ min⁻¹, more preferably higher then 1000 mM⁻¹ min⁻¹, most preferably higher then 4000 mM⁻¹ min⁻¹. Similarly, the Michaelis constant Kₘ of the modified GAPDH enzyme for NADP in the conversion reaction of glyceraldehyde 3-phosphate to 1,3-bisphosphoglycerate is lower than that of the unmodified enzyme. As the unmodified enzyme has a Kₘ value for NADP which regularly exceeds the measurable range, the desired Kₘ value for NADP is defined in absolute terms. For example, the Kₘ value is preferably lower than about 2 mM, more preferably lower than about 1 mM, more preferably lower than about 0.5 mM and most preferably lower than about 0.1 mM.

The modified GAPDH enzymes of the invention may still be able to bind and reduce the coenzyme NAD with a sufficiently high binding affinity. In a preferred embodiment of the invention, however, the specificity of the modified GAPDH for NADP⁺ is higher than its specificity for NAD. More preferably, the specificity as measured by constant k_{cat}/Kₘ of the modified GAPDH enzyme will be at least about 1x, 2x, 3x, 4x, 5x, 6x, 7x, 8x, 9x, 10x, 15x, 20x or more higher than that of the unmodified enzyme.

The modified enzymes that are provided by the present invention are intended for use in biotechnological production processes, e.g., the production of amino acids. It is therefore important to consider that the amino acid substitutions and deletions, either at positions relevant for the NADP specificity or not, do not result in a significant loss of activity of the enzyme due to a spatial rearrangement of the polypeptide structure. The modified GAPDH enzyme provided by the invention is enzymatically active, which means that it has retained at least part of the enzymatic activity of the GAPDH enzyme from which it was derived, e.g. the enzyme depicted in SEQ ID NO:1. Preferably, the modified GAPDH enzyme provided by the present invention has retained 50%, 60%, 70%, 80%, 90%, 95%, 96%, 97%, 98%, or 99% of the activity of the original GAPDH enzyme. The activity of the modified GAPDH enzyme can be determined by the assay which is described in Example 3.

According to a particularly preferred embodiment, the amino acid replacements that have been introduced in the course of the modification according to the invention have no influence on the enzymatic activity of the mutated GAPDH enzyme.

The present invention also provides a polynucleotide encoding a modified GAPDH enzyme with an increased specificity for the coenzyme NADP or an enzymatically active fragment thereof. The invention also refers to an expression vector, which comprises a polynucleotide coding for such a modified GAPDH enzyme or fragment thereof. As used herein, an expression vector refers to a polynucleotide construct, normally comprised of DNA, which allows for the genetic transfer, replication and expression of an inserted polynucleotide in a host cell, wherein said polynucleotide is not native to said host cell, or which is native to said host cell, but has been modified. Replication of the vector can be achieved either by integration of the vector into the genome of the host cell, or by episomal replication (e.g. plasmids). In the latter case, the expression vector comprises a functional origin of replication which allows the replication of the plasmid in the respective host cell.

The expression vector for use in the present methods is preferably a vector having a high copy number within the host cells so as to provide for high expression levels of the modified GAPDH enzyme in the host cell. The expression vector will normally also comprise a polynucleotide which encodes a selectable marker that is useful for identifying and selecting host cells which have been successfully transformed with the vector. Useful markers include those which confer resistance to antibiotics, such as ampicillin, kanamycin, and tetracycline. Such markers are known in the art, and the choice of an appropriate marker will depend on the host cell and the vector that is used.

Preferred expression vectors for use in the present invention comprise a polynucleotide of the invention in a form that allows for the expression of said polynucleotide in a host cell. Normally, the expression vector will include regulatory sequences, such as promoters and enhancers, which are operatively linked to the polynucleotide selected for expression. The promoter used in the expression vector may be a constitutive or an inducible promoter. It is appreciated that the design of the choice of the specific elements for inclusion into the expression vector will depend on the host cell to be transformed, the desired level of polypeptide expression, and the like. Preferably, the expression vectors are designed for the expression of a modified GAPDH enzyme of the invention in prokaryotic cells. More preferably, the expression vectors allow for the expression of the modified GAPDH enzyme of the present invention in bacterial cells, such as in cells of *E*. *coli* and *C*. *glutamicum.*

The expression vectors may also provide for the expression of the modified GAPDH enzyme in the form of a fusion polypeptide. A fusion polypeptide refers to a non-naturally occurring hybrid polypeptide comprising at least two different polypeptides or polypeptide fragments which do occur naturally associated to each other. Expression vectors which result in the expression of fusion polypeptides normally add several amino acids to the polypeptide to be expressed, either at the N-terminus or at the C-terminus. The additional amino acid may be, for example, helpful for enhancing the expression of the GAPDH enzyme in the host cell, or for purifying said polypeptide after expression.

Also provided are host cells which contain a polynucleotide or an expression vector of the present invention. Different host cells can be used for the production of the modified GAPDH enzyme of the invention from the expression vector. In general, both eukaryotic (e.g. yeasts or animal cells) and prokaryotic host cells can be used for recombinantly producing the modified GAPDH enzyme. However, it is preferred that the host cell is a prokaryotic cell. Bacterial cells are particularly preferred. For example, the host cells may be bacteria of the genera *Escherichia, Serratia, Brevibacterium* and *Corynebacterium,* as these bacterial hosts are frequently used in industrial production processes, such as for amino acid production. Host cells of the genera *Escherichia* and *Corynebacterium* are the most preferred host cells according to the present invention. Strains of *E*. *coli* that can be used, e.g., for the production of amino acids such as L-lysine or L-threonine, comprise K-12, JM109, GT3 and others.

The host cell which is used for the biotechnological production processes, such as the production of amino acids, may be a cell which naturally possesses all biosynthetic enzymes which are required for producing the desired product, e.g., L-lysine or L-threonine. For example, where a wild-type *C*. *glutamicum* cell comprising the gene depicted in SEQ ID NO:1 is used for producing the amino acid L-lysine, the product yield is restricted by the limited supply of NADPH. Specifically, in *C*. *glutamicum,* the synthesis of 1 mol of L-lysine from glucose requires 4 mol of NADPH. By transforming the cells with a polynucleotide coding for a modified GAPDH enzyme of the invention, the yield of L-lysine can be significantly increased, since the modified GAPDH has an increased specificity for the coenzyme NADP and thus produces reduced NADPH. Particularly high yields are achieved by use of a GAPDH enzyme which has been modified to achieve a complete reversal of the coenzyme specificity, e.g. a modified *C*. *glutamicum* GAPDH in which the exchanges Asp35Gly, Leu36Thr and Thr37Lys have been made. Alternatively, the host cell used for the production process may be a cell which does not contain a gene encoding GAPDH or which contains a gene coding for a non-functional GAPDH. In these cells, a successful transformation with a polynucleotide of the invention would be reflected by the fact that the cells exhibit a GAPDH activity after transformation.

Depending on the host organism used in the desired production process, a suitable expression vector will be selected. Examples for suitable vectors for polypeptide expression in *E*. *coli* cells comprise, for example, the vectors of the pBluescript series, the vectors of the pUC series, e.g. pUC18, pUC19, pBR322, pBR329, pQE70, pQE60, pQE-9, pNH8A, pNH16a, pNH18A, pNH46A, ptrc99a, pKK223-3, pKK233-3, pDR540, pRIT5, pLG338, pKC30, pHSG299, pHSG399, pRep4, pACYC177, pACYC184, pRSF1010, pBW22, and the like. Examples for expression vectors suitable for *C. glutamicum* comprise, for example, pAM330, pHM1519, pAJ655, pAJ611, pAJ1844, pCG11 pCG2, pCG4 the mobilizable *E. coli* or *C. glutamicum* shuttle vectors, e.g. pEC-XT99A, pEC-XC99E, pET-XK99E. Further examples for suitable vectors are described, e.g. in Kirchner et al., 2003, J. Biotechnol., 104:287-299, and in "Cloning Vectors" (Pouwels et al. (eds.) Elsevier, Amsterdam New York Oxford, 1985). The expression vector may be transformed into the host cell by any suitable method. The expression vector may be introduced into the host cell, e.g. by electroporation, microinjection, particle bombardement or by chemical methods such as calcium phosphate-mediated transformation, as described in Maniatis et al. 1982, Molecular Cloning, A Laboratory Manual, Cold Spring Harbor Laboratory.

In a further aspect, the invention relates to the use of any of the modified GAPDH enzymes, polynucleotides, expression vectors or host cells discussed herein for generating reduced NADPH coenzyme. The production of the reduced NADPH coenzyme preferably takes place intracellular, i.e. within a host cell that has been transformed with a polynucleotide that encodes the modified GAPDH. In a preferred aspect, the invention therefore relates to the use of any of the modified GAPDH enzymes, polynucleotides, expression vectors or host cells disclosed herein for the intracellular production of the reduced form of the NADP coenzyme. In a more preferred aspect, the invention relates to the use of the modified GAPDH enzymes, polynucleotides, expression vectors or host cells discussed herein for the biotechnological production of an amino acid or an intermediate of the amino acid biosynthesis, a sugar, a fatty acid, a nucleotide or a vitamin. The use of the enzymes, polynucleotides, expression vectors or host cells in the production of amino acids, such as L-lysine or L-threonine is particularly preferred.

In a further aspect, the invention relates to a method of producing L-lysine and/or L-threonine, comprising the steps of (i) culturing a host cell which is capable of producing L-lysine and/or L-threonine and comprises a polynucleotide coding for the modified GAPDH of the invention (or an expression vector comprising such polynucleotide) in a culture medium under conditions which allow the expression of the modified GAPDH enzyme of the invention, wherein said modified GAPDH has an increased specificity to coenzyme NADP relative to the corresponding naturally occurring enzyme and comprises (a) an amino acid sequence which is at least 50% identical to the amino acid sequence depicted in SEQ ID NO:1; and (b) amino acid replacements in positions that correspond to amino acids 35 and 36 of SEQ ID NO:1, and (ii) obtaining L-lysine or L-threonine from the culture medium.

Appropriate cell culture media are well known in the art for different kinds of host cells, in particular for bacteria such as *E*. *coli.* For example, strains of *E*. *coli* can be conveniently grown in MB media, LB media and BHI media. A suitable standard minimal media for *E*. *coli* is M9. The specific media for use in cultivation of a host cell transformed with a polynucleotide encoding the modified GAPDH of the invention will depend on the particular strain which is selected for the production process. The skilled person will have no problems to find appropriate media for a host cell based on the extensive literature available in the art.

Cultivation of the cells expressing the GAPDH enzyme of the invention can be performed in accordance with standard fermentation techniques. For example, the cells can be cultured in a batch or fed-batch process. Cultivation methods are generally described in Encyclopedia of Bioprocess Technology - Fermentation, Biocatalysis, and Bioseparation, Volumes 1-5, Flickinger, M.C., Drew, S.W. (eds.), 1999 John Wiley & Sons. Typical temperature conditions will be in the range of 20°C to 42°C, more commonly 30°C to 40°C, and preferably 35°C to 38°C. The culture broth will typically have a pH of between 6.5 and 9.0, more typically 7.0 to 8.0, e.g. 7.5. Fermentation of the culture will be continued for a time period ranging from several hours to several days. In particular, if the cultivation is performed as a batch process, the cultivation time normally ranges from about 12 hours to about 36 hours. When using a continuous process, fermentation times might be up to 21 days or longer.

The purification of the products, L-threonine and/or L-lysine, from the culture media can be achieved by use of commonly known methods, e.g. by use of ion-exchange resins as described in U.S. Patent No. 5,342,766.

Fig. 1 shows a multiple sequence alignment between NAD-dependent glyceraldehyde 3-phosphate dehydrogenase enzymes from different bacterial organisms.

### EXAMPLES

The following examples are provided for illustrating the invention and should not be construed to limit the invention to certain embodiments.

### Example 1: Site-directed mutagenesis of gapA of C. glutamicum

The *gapA* gene encoding the glyceraldehyde 3-phosphate dehydrogenase enzyme was amplified from chromosomal DNA of *C*. *glutamicum* (ATCC 13032) by PCR using the following primers (restriction sites are underlined):
5'- GAGCCTCATATGACCATTCGTGTTGGTATTAACG -3' (SEQ ID NO:2), and
5'- TGTAGTCGACGAGCTTGGAAGCTACGAGC -3' (SEQ ID NO:3).

The PCR reaction mixture contained:
0.5 µl *C*. *glutamicum* chromosomal DNA (0.1 mg/µl)
0.5 µl primer 1 (SEQ ID NO:2; 10 pmol/µl)
0.5 µl primer 2 (SEQ ID NO:3; 10 pmol/µl)
0.5 µl pfu polymerase (Fermentas Co, LTD, Germany)
5.0 µl 2 mM dNTP
5.0 µl 10 buffer (10x)
38.0 µl water

The following PCR program was used for amplification:
95°C 5 min
95°C 1 min
55°C 1 min
72°C 2 min (30 cycles from step 2 to step 4)
72°C 15 min
4°C 10 min

The amplified fragment was purified and digested with the restriction enzymes NdeI and SalI. Subsequently, the digested fragment was ligated into the vector pET-28a+ (Invitrogen GmbH, Germany) that had also been digested with NdeI and SalI. The recombinant plasmid, designated pET-gapA, was transformed into *E. coli* JM109. The recombinant plasmid was purified and the nucleotide sequence was confirmed by sequencing.

The plasmid pET-gapA was used as a template for subsequent site-directed mutagenesis experiments which introduced mutations into the amino acid sequence of gapA. The mutagenesis experiments were carried out according to the instruction of QuickChangeII XL site-directed mutagenesis kit (Stratagene).

For the mutation of Asp at amino acid position 35 to Gly, the following primers were used:
5'-GTAGTTGCAGTCAACGGCCTCACCGACAACAAG-3' (SEQ ID NO:4) and
5'-CTTGTTGTCGGTGAGGCCGTTGACTGCAACTAC-3' (SEQ ID NO:5).

The mutant plasmid resulting from the PCR amplification reaction using the primers of SEQ ID NO:4 and 5 was designated pET-gapA(D35G).

For the double mutations of Asp at amino acid position 35 to Gly and Leu at amino acid position 36 to Thr, the following primers were used:
5'-GAGGTAGTTGCAGTCAACGGCACCACCGACAACAAGACCCTT-3'(SEQ ID NO:6)
5'-AAGGGTCTTGTTGTCGGTGGTGCCGTTGACTGCAACTACCTC-3'(SEQ ID NO:7).

The mutant plasmid resulting from the PCR amplification reaction using the primers of SEQ ID NO: 6 and 7 was designated pET-gapA(D35G/L36T).

For the double mutations Asp at amino acid position 35 to Gly and Leu at amino acid position 36 to Arg, a PCR was conducted by using the following oligonucleotide primers:
5'-GTAGTTGCAGTCAACGGCCGCACCGACAACAAGACC-3' and (SEQ ID NO:8)
5'-GGTCTTGTTGTCGGTGCGGCCGTTGACTGCAACTAC-3' (SEQ ID NO:9)

The plasmid resulting from the PCR amplification reaction using the primers of SEQ ID NO:8 and 9 was pET-gapA(D35G/L36R).

For the triple mutations of Asp at amino acid position 35 to Gly, Leu at amino acid position 36 to Thr, and Thr at amino acid position 37 to Lys, a PCR was conducted by using the following oligonucleotide primers:
5'-CTCGAGGTAGTTGCAGTCAACGGCACCAAGGACAACAAGACCCTTTCCACCC-3' (SEQ ID NO:10), and
5'-GGGTGGAAAGGGTCTTGTTGTCCTTGGTGCCGTTGACTGCAACTACCTCGAG-3' (SEQ ID NO:11)

The plasmid resulting from the PCR amplification reaction using the primers of SEQ ID NO:10 and 11 was pET-gapA(D35G/L36T/T37K).

For the triple mutations of Asp at amino acid position 35 to Gly, Leu at amino acid position 36 to Thr, and Pro at amino acid position 192 to Ser, plasmid pET-gapA(D35G/L36T) was used as template and a PCR was conducted by using the following oligonucleotide primers:
5'-CAGCGCCTGCACGATGCAAGTCACCGCGAC-3' (SEQ ID NO:12), and
5'- GTCGCGGTGACTTGCATCGTGCAGGCGCTG -3' (SEQ ID NO:13)

The plasmid resulting from the above PCR amplification reaction was pET-gapA(D35G/L36T/P192S).

For the triple mutations of Asp at amino acid position 35 to Gly, Leu at amino acid position 36 to Arg, and Pro at amino acid position 192 to Ser, plasmid pET-gapA(D35G/L36R) was used as template and a PCR was conducted by using oligonucleotide primers of SEQ ID NO:12 and 13. The resulting plasmid was designated as pET-gapA(D35G/L36R/P192S).

For the four-points mutations of Asp at amino acid position 35 to Gly, Leu at amino acid position 36 to Thr, Thr at amino acid position 37 to Lys, and Pro at amino acid position 192 to Ser, plasmid pET-gapA(D35G/L36T/T37K) was used as template and a PCR was conducted by using oligonucleotide primers of SEQ ID NO:12 and 13. The resulting plasmid was designated as pET-gapA(D35G/L36T/T37K/P192S).

The nucleotide sequence of all plasmids encoding a mutated amino acid sequence of the glyceraldehyde-3-phosphate dehydrogenase enzyme of *C*. *glutamicum* was confirmed by sequencing. The plasmids were stored at -80°C or directly used for the subsequent transformation experiments.

### Example 2: Expression of mutated glyceraldehyde-3-phosphate dehydrogenase enzymes

The plasmid pET-gapA and the plasmids encoding the mutated glyceraldehyde-3-phosphate dehydrogenase genes prepared in accordance with Example 1, were transformed into the *E. coli* strain BL21 (DE3) CodonPlus RIPL (Stratagene, Germany) by heat-shock according to common protocols. The recombinant *E. coli* strains resulting from the transformation experiments were designated:
*E. coli* gapA(D35G),
*E. coli* gapA(D35G/L36T),
*E. coli* gapA(D35G/L36R),
*E. coli* gapA(D35G/L36T/T37K),
*E. coli* gapA(D35G/L36T/P192S),
*E. coli* gapA(D35G/L36R/P192S), and
*E. coli* gapA(D35G/L36T/T37K/P192S).

The above recombinant *E. coli* strains were cultured at 37°C in LB medium containing 100 mg/L kanamycine until an OD₆₀₀ of 0.6 was reached. Then, (isopropyl-beta-D-thiogalactopyranoside (IPTG) was added to the medium to a final concentration of 0.1 mM, and the bacteria were cultured at 20°C for another 12-14 h. The *E. coli* cells were then harvested and washed and suspended in the buffer of 50 mM Na₂HPO₄ (pH 7.5), 0.2 mM EDTA and 0.1 mM dithiothreitol. Suspended cells were disrupted by sonication and centrifuged at 13.000 rpm for 1 h. The supernatant was purified by use of a Ni²⁺ NTA column (GE Healthcare Bio-Sciences, Piscataway, NJ).

### Example 3: Measuring the enzyme activity of purified glyceraldehyde-3-phosphate dehydrogenase enzyme

The enzyme activity of glyceraldehyde-3-phosphate dehydrogenase was determined by monitoring the change of NAD(P)H in absorbance at 340 nm at 25°C using a Multiskan® spectrophotometer (Thermo Scientific, Germany). Initial rate measurements were carried out in 50 mM triethanolamine hydrochloride buffer (pH 8.5), 0.2 mM EDTA, 50 mM Na₂HPO₄, 1.5 mM glyceraldehyde-3-phosphate (Clermont et al., 1993). When saturation could be reached, kinetic parameters were obtained from activity measurements with coenzyme concentrations ranging from 0.1 Kₘ to 10 Kₘ. Each rate measurement was run in triplicate. One unit of enzyme activity corresponds to one µmol of NAD(P)H formed per minute. Kinetic constants were obtained from non-linear regression data analysis and expressed as mean ± S.D.

The kinetic parameters determined for wild-type and mutated forms of glyceraldehyde-3-phosphate dehydrogenase are shown in Table 1. It can be seen that coenzyme specificity of glyceraldehyde-3-phosphate has been changed from NAD to NADP by the introduction of mutations at amino positions 35, 36, 37 and 192. More specifically, mutants comprising amino acid exchanges D35G/L36T/T37K, D35G/L36T/P192S, D35G/L36R/P192S, or D35G/L36T/T37K/P192S show a high catalytic efficiency and can utilize both NAD⁺ and NADP⁺.

**Table 1: Kinetic parameters of wild-type and mutated glyceraldehyde-3-phosphate dehydrogenase**

| **Enzyme** | **Coenzyme** | ***Kₘ [mM]*** | ***K_{cat} [min⁻¹]*** | ***K_{cat}*/*Kₘ [mM min⁻¹]*** |
|---|---|---|---|---|
| Wild-type | + NADP⁺ | ND | ND | ND |
| | NAD⁺ | 0.048 ± 0.002 | 387 ± 28 | 8063 ± 540 |
| D35G | NADP⁺ | 0.83 ± 0.12 | 178 ± 11 | 215 ± 22 |
| | NAD⁺ | 0.54 ± 0.07 | 382 ± 32 | 707 ± 82 |
| D35G/L36T | NADP⁺ | 0.28 ± 0.016 | 352 ± 52 | 1257 ± 118 |
| | NAD⁺ | 0.22 ± 0.038 | 286 ± 37 | 1300 ± 154 |
| D35G/L36R | NADP⁺ | 0.31 ± 0.034 | 348 ± 26 | 1123 ± 176 |
| | NAD⁺ | 0.18 ± 0.017 | 204 ± 22 | 1134 ± 132 |
| D35G/L36T/T37K | NADP⁺ | 0.10 ± 0.012 | 480 ± 36 | 4800 ± 232 |
| | NAD⁺ | 0.47 ± 0.032 | 252 ± 33 | 536 ± 48 |
| D35G/L36T/Pl92S | NADP⁺ | 0.070 ± 0.003 | 395 ± 28 | 4356 ± 215 |
| | NAD⁺ | 0.12 ± 0.004 | 227 ± 17 | 1952 ± 113 |
| D35G/L36R/Pl92S | NADP⁺ | 0.10 ± 0.012 | 389 ± 37 | 3885 ± 247 |
| | NAD⁺ | 0.058 ± 0.002 | 172 ± 11 | 2954 ± 228 |
| D35G/L36T/T37K/PI92S | NADP⁺ | 0.070 ± 0.003 | 428 ± 45 | 5868 ± 352 |
| | NAD⁺ | 0.10 ± 0.014 | 164 ± 12 | 1640 ± 108 |

### Example 4: Integration of gapA mutations D35G/L36T/T37K into the chromosome of a Corynebacterium glutamicum strain having an aspartokinase mutation lysC(Q298A)

To construct a plasmid-free mutant, homologous recombination was carried out to integrate the *gapA* point mutations D35G/L36T/T37K into the chromosomal DNA of the lysine producing *C*. *glutamicum* strain lysC(Q298A). The construction of the strain lysC(Q298A) has been described in detail in co-pending European patent application 10003770.4. This strain has a point mutation in the gene encoding the aspartokinase III enzyme and is significantly less sensitive towards inhibition by lysine and threonine than the wild-type *C*. *glutamicum.*

A cassette for the homologous recombination was amplified by PCR using genomic DNA of *C*. *glutamicum* ATCC 13032 as a template and the following primers:
5'-ACTCGTCTAGAGTTGGTAGGGAGGCAATGAT-3' (SEQ ID NO:14)
5'-TCTGAGGATCCACATGTGGTTAAAGGTCTAGCG-3' (SEQ ID NO:15)

The amplified fragment was purified, digested with XbaI and *BamHI,* purified again and ligated into a pUC18 vector which had been linearized with the same restriction enzymes. The recombinant plasmid was designated pUC18-gapA. The integrity of the insert was confirmed by sequencing. pUC18-gapA was used as a template for subsequent mutagenesis experiments which introduced site-directed mutations into the amino acid sequence of gapA at a predicted position. The mutagenesis experiments were carried out according to the instruction of QuickChangeII XL site-directed mutagenesis kit (Stratagene, Germany).

The plasmid resulting from the PCR amplification reaction using the primers of SEQ ID NO:10 and 11 was designated pUC18-gapA(D35G/L36T/T37K) and its nucleotide sequence was confirmed by sequencing. Plasmid pUC18-gapA(D35G/L36T/T37K) was digested with *BamHI* and *XbaI*. The fragment of gapA(D35G/L36T/T37K) obtained by restriction was subcloned into pK18mobsacB vector (Kirchner et al. (2003), J. Biotechnol. 104: 287-299) previously digested with *Bam*HI-*Xba*I, and transformed into competent cells of *E. coli DB5α-mcr* (Grant et al. (1990), Proc. Natl. Acad. Sci. USA. 87:4645-4649). The resulting recombinant plasmid was purified and is referred to as pKl8mobsacB-gapA(D35G/L36T/T37K). This plasmid was subsequently introduced into the lysine producing strain *C. glutamicum* strain lysC(Q298A) by electroporation (Van der Rest et al. (1999), Appl. Microbiol. Biotechnol. 52, 541-545). The vector cannot replicate independently in this strain and is only retained in the cell when integrated in the chromosome as the result of a recombination event.

Clones which successfully integrated the plasmid into the genome by a first homologous recombination event were selected by cultivation on LBHIS medium containing 15 mg/l kanamycin at 30°C for 48 hours. Incubation of a positive clone in kanamycin-free LBHIS medium allows a second recombination event. Clones in which the vector backbone has been successfully eliminated by a second recombination event were identified by growth on sucrose-containing medium. Only clones which have lost the vector backbone comprising the *SacB* gene (encoding levansucrase) will survive; the *SacB* gene can be used as a positive selection marker as cells which carry this gene cannot grow on sucrose-containing medium. Depending on the location of the second recombination event in relation to the mutation site, allele replacement or incorporation of the mutation takes place in the excision or the original copy remains in the chromosome of the host.

Clones in which the two recombination events have led to successful replacement of the native gapA-coding region were identified by sequencing of a PCR-product spanning the relevant region. The PCR product was generated using genomic DNA of individual clones as a template and the primers depicted in SEQ ID NOs:14 and 15. The PCR-product was purified and sequenced. PCR products with positive integration were stored. The mutant strain is referred to as *C*. *glutamicum* lysC(Q298A)gapA(D35G/L36T/T37K).

### Example 5: Production of lysine by the mutant C. glutamicum lysC(Q298A)gapA(D35G/L36T/T37K)

The recombinant strains of *C. glutamicum* lysC(Q298A)gapA (D35G/L36T/T37K) and *C*. *glutamicum* lysC(Q298A) as well as the wild-type strain *C*. *glutamicum* ATCC 13032 were cultured in shaking flasks to determine the production of L-lysine. The fermentation medium was composed of CSL 5 g/L, MOPO 20 g/L, 100 g/L glucose, 25 g/L (NH₄)₂SO₄, 0.1 g/L KH₂PO₄, 1 g/L MgSO₉•7H₂O, 0.01 g/L FeSO₄•7H₂O, 0.005 g/L MnSO₄•5H₂O, 0.01 g/L CaCl₂•2H₂O, 0.3 mg/L Biotin, 0.3 mg/L Thiamine-HCl, 25 g/L CaCO₃. All three strains were cultured at 32°C.

The production of L-lysine by the *C. glutamicum* strains were monitored by HPLC. The yields of L-lysine determined in this manner are summarized in Table 2 below. For the recombinant strains L-Lysine concentrations after the consumption of glucose are included. It is shown that the introduction of a glyceraldehyde-3-phosphate dehydrogenase with altered coenzyme specificity to NADP significantly improved the yield of lysine.

**Table 2: Production of lysine in C. glutamicum**

| **Bacterial strain** | **L-lysine production (g/L)** |
|---|---|
| *C. glutamicum* ATCC 13032 | 0 |
| *C. glutamicum* lysC(Q298A) | 9.0 |
| *C. glutamicum* lysC(Q298A)gapA(D35G/L36T/T37K) | 11.5 |

## Claims

1. Modified glyceraldehyde-3-phosphate dehydrogenase (GAPDH) enzyme, comprising
(a) an amino acid sequence which is at least 50% identical to the amino acid sequence depicted in SEQ ID NO:1; and
(b) amino acid replacements in positions that correspond to amino acids 35 and 36 of SEQ ID NO:1;
wherein said modified enzyme has an increased specificity to coenzyme NADP relative to the corresponding naturally occurring enzyme.

2. Modified GAPDH enzyme of claim 1, wherein
(a) Asp in the position that corresponds to amino acid 35 of SEQ ID NO:1 has been replaced by Gly, Ala, Val, Leu, Ile, or Pro; and
(b) Leu in the position that corresponds to amino acid 36 of SEQ ID NO:1 has been replaced by Ser, Thr, Arg, His or Lys.

3. Modified GAPDH enzyme of claim 1 or 2, wherein Asp in the position that corresponds to amino acid 35 of SEQ ID NO:1 has been replaced by Gly, and Leu in the position that corresponds to amino acid 36 of SEQ ID NO:1 has been replaced by Thr.

4. Modified GAPDH enzyme of any of claims 1-3, further comprising one or more of the following replacements:
(a) Thr or Leu in the position that corresponds to amino acid 37 of SEQ ID NO:1 has been replaced by Lys, Arg or His;
(b) Pro in the position that corresponds to amino acid 192 of SEQ ID NO:1 has been replaced by Ser, Thr or Arg.

5. Modified GAPDH enzyme of any of claims 1-4, which comprises a combination of amino acid exchanges selected from the group of:
(a) Asp in the position that corresponds to amino acid 35 of SEQ ID NO:1 has been replaced by Gly; Leu in the position that corresponds to amino acid 36 of SEQ ID NO:1 has been replaced by Thr; and Thr or Leu in the position that corresponds to amino acid 37 of SEQ ID NO:1 has been replaced by Lys;
(b) Asp in the position that corresponds to amino acid 35 of SEQ ID NO:1 has been replaced by Gly; Leu in the position that corresponds to amino acid 36 of SEQ ID NO:1 has been replaced by Thr; and Pro in the position that corresponds to amino acid 192 of SEQ ID NO:1 has been replaced by Ser;
(c) Asp in the position that corresponds to amino acid 35 of SEQ ID NO:1 has been replaced by Gly; Leu in the position that corresponds to amino acid 36 of SEQ ID NO:1 has been replaced by Thr; and Thr or Leu in the position that corresponds to amino acid 37 of SEQ ID NO:1 has been replaced by Lys; and Pro in the position that corresponds to amino acid 192 of SEQ ID NO:1 has been replaced by Ser.
(d) Asp in the position that corresponds to amino acid 35 of SEQ ID NO:1 has been replaced by Gly; Leu in the position that corresponds to amino acid 36 of SEQ ID NO:1 has been replaced by Arg; and Thr or Leu in the position that corresponds to amino acid 37 of SEQ ID NO:1 has been replaced by Lys;
(e) Asp in the position that corresponds to amino acid 35 of SEQ ID NO:1 has been replaced by Gly; Leu in the position that corresponds to amino acid 36 of SEQ ID NO:1 has been replaced by Arg; and Pro in the position that corresponds to amino acid 192 of SEQ ID NO:1 has been replaced by Ser; and
(f) Asp in the position that corresponds to amino acid 35 of SEQ ID NO:1 has been replaced by Gly; Leu in the position that corresponds to amino acid 36 of SEQ ID NO:1 has been replaced by Arg; and Thr or Leu in the position that corresponds to amino acid 37 of SEQ ID NO:1 has been replaced by Lys; and Pro in the position that corresponds to amino acid 192 of SEQ ID NO:1 has been replaced by Ser.

6. Polynucleotide encoding a modified GAPDH enzyme of any of claims 1-5.

7. Expression vector comprising the polynucleotide of claim 6.

8. Host cell comprising the polynucleotide of claim 6 or the expression vector of claim 7.

9. Host cell of claim 8, wherein the host cell is a prokaryotic cell.

10. Host cell of claim 9, wherein the host cell is a bacterial cell.

11. Host cell of claim 10, wherein the bacterial cell is an *Escherichia* cell.

12. Host cell of claim 10, wherein the bacterial cell is a *Corynebacterium* cell.

13. Use of a modified GAPDH enzyme of any of claims 1 to 5, a polynucleotide of claim 6, an expression vector of claim 7 or a host cell of claims 8-12, for the intracellular production of the reduced form of the NADPH coenzyme.

14. Use of a modified GAPDH enzyme of any of claims 1 to 5, a polynucleotide of claim 6, an expression vector of claim 7 or a host cell of claims 8-12, for the biotechnological production of an amino acid, an intermediate of the amino acid biosynthesis, sugar, fatty acid, nucleotide or vitamin.

15. Method of producing L-lysine and/or L-threonine, comprising the steps of (i) culturing a host cell of any of claims 8-10, which is capable of producing L-lysine and/or L-threonine, in a culture medium under conditions which allow the expression of the modified GAPDH enzyme of any of claims 1 to 5, and (ii) obtaining L-lysine and/or L-threonine from the culture medium.


SEQUENCE LISTING

<110>  Technische Universität Hamburg-Harburg

<120>  MODIFIED GLYCERALDEHYDE-3-PHOSPHATE DEHYDROGENASE FROM
       CORYNEBACTERIUM GLUTAMICUM AND USES THEREOF

<130>  P 85641

<160>  17

<170>  PatentIn version 3.3

<210>  1
<211>  334
<212>  PRT
<213>  Corynebacterium glutamicum

<400>  1

Met Thr Ile Arg Val Gly Ile Asn Gly Phe Gly Arg Ile Gly Arg Asn
1               5                   10                  15

Phe Phe Arg Ala Val Leu Glu Arg Ser Asp Asp Leu Glu Val Val Ala
            20                  25                  30

Val Asn Asp Leu Thr Asp Asn Lys Thr Leu Ser Thr Leu Leu Lys Phe
        35                  40                  45

Asp Ser Ile Met Gly Arg Leu Gly Gln Glu Val Glu Tyr Asp Asp Asp
    50                  55                  60

Ser Ile Thr Val Gly Gly Lys Arg Ile Ala Val Tyr Ala Glu Arg Asp
65                  70                  75                  80

Pro Lys Asn Leu Asp Trp Ala Ala His Asn Val Asp Ile Val Ile Glu
                85                  90                  95

Ser Thr Gly Phe Phe Thr Asp Ala Asn Ala Ala Lys Ala His Ile Glu
            100                 105                 110

Ala Gly Ala Lys Lys Val Ile Ile Ser Ala Pro Ala Ser Asn Glu Asp
        115                 120                 125

Ala Thr Phe Val Tyr Gly Val Asn His Glu Ser Tyr Asp Pro Glu Asn
    130                 135                 140

His Asn Val Ile Ser Gly Ala Ser Cys Thr Thr Asn Cys Leu Ala Pro
145                 150                 155                 160

Met Ala Lys Val Leu Asn Asp Lys Phe Gly Ile Glu Asn Gly Leu Met
                165                 170                 175

Thr Thr Val His Ala Tyr Thr Gly Asp Gln Arg Leu His Asp Ala Pro

180                 185                 190

His Gly Asp Leu Arg Arg Ala Arg Ala Ala Ala Val Asn Ile Val Pro
        195                 200                 205

Thr Ser Thr Gly Ala Ala Lys Ala Val Ala Leu Val Leu Pro Glu Leu
    210                 215                 220

Lys Gly Lys Leu Asp Gly Tyr Ala Leu Arg Val Pro Val Ile Thr Gly
225                 230                 235                 240

Ser Ala Thr Asp Leu Thr Phe Asn Thr Lys Ser Glu Val Thr Val Glu
                245                 250                 255

Ser Ile Asn Ala Ala Ile Lys Glu Ala Ala Val Gly Glu Phe Gly Glu
            260                 265                 270

Thr Leu Ala Tyr Ser Glu Glu Pro Leu Val Ser Thr Asp Ile Val His
        275                 280                 285

Asp Ser His Gly Ser Ile Phe Asp Ala Gly Leu Thr Lys Val Ser Gly
    290                 295                 300

Asn Thr Val Lys Val Val Ser Trp Tyr Asp Asn Glu Trp Gly Tyr Thr
305                 310                 315                 320

Cys Gln Leu Leu Arg Leu Thr Glu Leu Val Ala Ser Lys Leu
                325                 330

<210>  2
<211>  34
<212>  DNA
<213>  artificial

<220>
<223>  oligonucleotide primer

<400>  2
gagcctcata tgaccattcg tgttggtatt aacg                                34

<210>  3
<211>  29
<212>  DNA
<213>  artificial

<220>
<223>  oligonucleotide primer

<400>  3
tgtagtcgac gagcttggaa gctacgagc                                      29

<210>  4
<211>  33

<212>  DNA
<213>  artificial

<220>
<223>  oligoncleotide primer

<400>  4
gtagttgcag tcaacggcct caccgacaac aag                                    33


<210>  5
<211>  33
<212>  DNA
<213>  artificial

<220>
<223>  oligonucleotide primer

<400>  5
cttgttgtcg gtgaggccgt tgactgcaac tac                                    33


<210>  6
<211>  42
<212>  DNA
<213>  artificial

<220>
<223>  oligonucleotide primer

<400>  6
gaggtagttg cagtcaacgg caccaccgac aacaagaccc tt                          42


<210>  7
<211>  42
<212>  DNA
<213>  artificial

<220>
<223>  oligonucleotide primer

<400>  7
aagggtcttg ttgtcggtgg tgccgttgac tgcaactacc tc                          42


<210>  8
<211>  36
<212>  DNA
<213>  artificial

<220>
<223>  oligonucleotide primer

<400>  8
gtagttgcag tcaacggccg caccgacaac aagacc                                 36


<210>  9
<211>  36
<212>  DNA
<213>  artificial

<220>
<223>  oligonucleotide primer

<400>  9
ggtcttgttg tcggtgcggc cgttgactgc aactac                                36


<210>  10
<211>  52
<212>  DNA
<213>  artificial

<220>
<223>  oligonucleotide primer

<400>  10
ctcgaggtag ttgcagtcaa cggcaccaag gacaacaaga ccctttccac cc              52


<210>  11
<211>  52
<212>  DNA
<213>  artificial

<220>
<223>  oligonucleotide primer

<400>  11
gggtggaaag ggtcttgttg tccttggtgc cgttgactgc aactacctcg ag              52


<210>  12
<211>  30
<212>  DNA
<213>  artificial

<220>
<223>  oligonucleotide primer

<400>  12
cagcgcctgc acgatgcaag tcaccgcgac                                       30


<210>  13
<211>  30
<212>  DNA
<213>  artificial

<220>
<223>  oligonucleotide primer

<400>  13
gtcgcggtga cttgcatcgt gcaggcgctg                                       30


<210>  14
<211>  31
<212>  DNA
<213>  artificial

<220>
<223>  oligonucleotide primer

<400>  14
actcgtctag agttggtagg gaggcaatga t                                     31

<210>  15
<211>  33
<212>  DNA
<213>  artificial

<220>
<223>  oligonucleotide primer

<400>  15
tctgaggatc cacatgtggt taaaggtcta gcg                                    33


<210>  16
<211>  5
<212>  PRT
<213>  Artificial

<220>
<223>  Phosphate binding site consensus sequence


<220>
<221>  VARIANT
<222>  (4)..(4)
<223>  Thr can be replced by Leu in some organisms

<400>  16

Asn Asp Leu Thr Asp
1               5


<210>  17
<211>  6
<212>  PRT
<213>  Corynebacterium glutamicum


<220>
<221>  VARIANT
<222>  (2)..(2)
<223>  Xaa can be any amino acid

<220>
<221>  VARIANT
<222>  (4)..(4)
<223>  Xaa can be any amino acid

<220>
<221>  VARIANT
<222>  (5)..(5)
<223>  Xaa can be any amino acid

<400>  17

Gly Xaa Gly Xaa Xaa Gly
1               5